# EUROPEAN PATENT APPLICATION

(11) **EP 1 159 989 A1**
(43) Date of publication of application: **05.12.2001**
(21) Application number: 00201856.2
(22) Date of filing: 24.05.2000
(51) Int. Cl.: A63B 24/00

(54) **A method of generating and/or adjusting a training schedule**

(71) Applicant: In2Sports B.V., 5612 AR Eindhoven (NL)
(72) Inventor: de Vries, Govert, 5616 BE Eindhoven (NL); Boddeke, Frank Robbert, Emerald Hills, CA 94062 (US); Oosterholt, Ronaldus Hermanus Theodorus, 6006 HG Weert (NL); Boddeke-de Jong, Hiske Aldine, Emerald Hills, CA 94062 (US)
(74) Representative: Aalbers, Arnt Reinier

(57) **Abstract**

The invention pertains to a method of generating and/or adjusting a training schedule based on personal data and optionally on preferences of a particular user. The method involves electronically obtaining one or more parameters from and/or concerning the user during a training session preferably by means of at least one sensor, selecting and obtaining a training schedule from a database and/or adjusting an earlier selected schedule in accordance with the said obtained parameters. The database may be improved on the basis of the parameters obtained from and/or the training schedules adjusted for a plurality of users.

## Description

The invention pertains to a method of generating a training schedule based on personal data and optionally preferences, such as goal and frequency of the exercises, of a particular user. The invention further relates to a computer program product, which is optionally stored on a computer readable storage medium, to a portable device, and to a server system.

Many amateur and professional athletes establish a training schedule by conferring with a coach so as to ensure that they train as efficiently and effectively as possible, i.e. to ensure that they do not train too much (thus increasing the risk of receiving an injury) or too little.

However, such coaches are relatively expensive and only available to a limited extent.

It is an object of the present invention to provide a method of the above-mentioned type, wherein personal coaching is readily available to a large number of athletes and at relatively low costs.

To this end, the invention provides a method of generating and/or adjusting a training schedule based on personal data and optionally preferences of a particular user which method involves electronically obtaining one or more parameters from and/or concerning the user during a training session preferably by means of at least one sensor, selecting and obtaining a training schedule from a database and/or adjusting an earlier selected schedule in accordance with the said obtained parameters.

Thus personalised training schedules, based on individual parameters of a particular user that are obtained during a training session, can be generated and/or adjusted automatically. The said parameters may be provided by the user him or herself, for example by means of e.g. a small keyboard on a portable computer device worn by the user, or they may be obtained automatically by means of at least one sensor.

It is preferred that a plurality of sensors is used. Further it is preferred that, in case of the user being a walker or runner, the said parameters relate at least to the heart rate and the distance covered during a training session, since these parameters are most pertinent to the performance of the user during a training session. For a tennis player, such parameters may relate to e.g. the number of backhands performed and the acceleration of the racket.

The obtained parameters can be uploaded, e.g. by means of a mobile phone using a Wireless Application Protocol, Universal Mobile Telecommunications System, General Packet Radio Service or the like via the Internet, to a remote service system maintained by e.g. a service provider, which service system selects and obtains a training schedule and/or adjusts an earlier selected schedule in accordance with the said parameters, and wherein the said schedule is subsequently downloaded.

The database or specific components of the database, such as the schedules contained in the database or the rules that govern selection and/or adjustment of the schedules, can be improved on the basis of parameters obtained from and/or the training schedules adjusted for a plurality of users. Thus, future selections from the database and/or adjustments can be improved based on e.g. the efficacy (derived from the progress of the said users) of earlier selections and/or adjustments. Also, the database may be arranged to recognise an increased risk of injury based on past experiences and issue warnings accordingly.

The invention also relates to a computer program product which carries out the method steps of any one of the methods described above and to a computer program product stored on a computer readable storage medium comprising code means adapted to carry out the method steps of the method described above. The said computer program product may of course comprise both modules intended for implementation on a remote service system, e.g. located at a service provider, and modules intended for implementation on a local processing unit, such as a personal computer and/or portable computer device of some sort.

The invention further pertains to a portable device at least comprising an input device, such as preferably one or more sensors, for obtaining one or more parameters from and/or concerning a user during a training session, a storage medium, e.g. a Random Access Memory or the like, a controller adapted to store a training schedule and connected to the input device and the storage medium, the controller being operative to perform at least the following functions: receive the one or more parameters from the input device, and store the one or more parameters in the storage medium. The portable device further comprises an interface for transferring at least some of the stored information to a computer that contains or is connected or connectable to a database of training schedules. The said portable device is preferably provided with a display and may be worn e.g. around the wrist of the user.

The invention also relates to a server system for generating and/or adjusting a training schedule based on personal data and optionally preferences of a particular user, an interface for receiving parameters from and/or concerning the user obtained during a training session, a storage medium for the said parameters, and a component for selecting and obtaining a training schedule from a database and/or adjusting an earlier selected schedule in accordance with the said parameters.

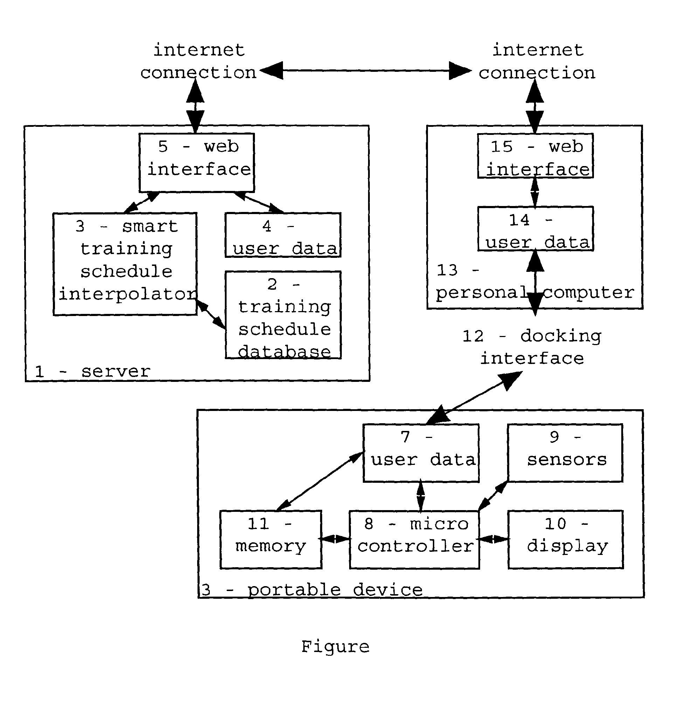

The invention will be further explained by reference to the figure in which a preferred embodiment of the method of the invention is schematically shown. It will be understood that the method of the invention is not in any way restricted to this specific and preferred embodiment.

The figure shows a system in which a method according to the invention involving remote selection and/or adjustment of a training schedule may be implemented.

The said system comprises a server 1, which in turn comprises a training schedule database 2, a smart training schedule interpolator 3 for selecting and obtaining a training schedule from the database 2, a storage medium 4 for storing user data, and a web interface 5.

The web interface 5 can be accessed via the Internet by the user with a web browser, such as Microsoft Explorer or Netscape Navigator or similar (future) means. The said web interface 5 has the following main tasks: receiving information that is being uploaded by the user and/or that was obtained directly from the user, e.g. by means of an electronic questionnaire, and during a training session by means of a portable device that will be discussed hereinafter; downloading personalised training schedules to the user and optionally digital sound files or additional information, such as general information concerning exercises, nutrition, health issues, or an extensive analysis of the performance and/or progress of the user; and storing information obtained from the user as well as information generated for the user in the storage medium 4. The web interface 5 may also be arranged to download information concerning (a particular group of) other users, such as average performance or "high scores", or advertisements.

The database 2 contains training schedules which are designed by experts, such as professional athletes and coaches and may concern specific exercises and information relating to the duration, intensity and frequency of these exercises. For instance, a runner could be instructed to run a track of five miles thrice a week for a period of two months or to run a track of three miles daily for a period of two weeks whilst performing twenty-five push-ups after each mile.

The smart training schedule interpolator 3 generates and/or adjusts personalised training schedules by electronically selecting and obtaining a training schedule from the database 2 and/or adjusting an earlier selected schedule in accordance with the said information concerning the user. The interpolator 3 may be monitored by e.g. a professional coach that assists and instructs the interpolator 3 in unusual cases.

Information from and/or concerning the user may be obtained during a training session by means of a portable device 6. The portable device 6 that is shown in the figure comprises a storage medium 7 for storing user data, a plurality of sensors 9, a display 10, and a further memory 11 for e.g. storing one or more training schedules and optionally one or more digital sound files containing music or spoken instructions in a format such as MP3, MIDI, or the like. All of these components are operatively connected to a micro-controller 8. Further, as will be appreciated by the skilled person, the storage medium 7 and the memory 11 may be integrated into a single storage medium.

The sensor or sensors 9 may e.g. consist of or comprise a probe that measures electrical pulses generated by the heart of the user, one or more accelerometers (preferably two or more orthogonal accelerometers) for measuring the acceleration of at least one part of the body of the user, such as the waist or wrist, or e.g. a temperature or pressure probe for respectively measuring the temperature of at least one part of the body or the blood pressure of the user. The sensors or sensors 9 also may be used to measure certain parameters, such as acceleration(s) of sport equipment such as a tennis racket, a base ball bat, a hockey stick, or a javelin so as establish e.g. the efficacy of a certain movement or the likelihood of injuries.

As an alternative or in addition to the sensors 9, another input device, such as a small keyboard, a touch-pad or a microphone, may be provided so as to enable the user to feed the micro-controller 8 with (additional) data.

The display 10 may be arranged and used for showing a training schedule and exercise progress and analysis. The display 10 may comprise a touch-screen, thus integrating the above-mentioned input device in the display 10.

The micro-controller 8 collects data from the sensors 9 or the input device and drives the display 10. It further stores and retrieves data in respectively from the mentioned storage media 7, 11.

The portable device 6 may comprise a transmitter or a wireless modem which enables it to communicate directly with the server 1 or with an intermediate computer. Thus, further training schedules and/or digital sound files can be downloaded during a training session. Also, the user can send a distress signal, which notifies e.g. family or a hospital, or position data with which the position of the user can be plotted on an electronic map generated by the web interface 5 of the server 1.

Alternatively or additionally, the portable device 6 may comprise a connector (not shown), which, upon placing the portable device 6 into the cradle of a docking interface 12 comprising a connector-counterpart, enables data exchange of user data on the one hand and generated and/or adjusted training schedules and digital sound files on the other between the portable device 6 and a personal computer 13. The said cradle may further comprise a contact element for enabling power transmission to the portable device 6, for instance to recharge a battery contained in the said portable device 6.

A suitable configuration for the mention personal computer 13 will be described hereinafter. However, it preferably at least comprises a storage medium 14 for storing user data and a web interface and preferably also a storage medium for storing training schedules, digital sound files and the like.

As will be clear from the above-described examples, the present invention can provide personalised coaching to a virtually unlimited number of people at relatively low costs.

It will also be clear that the present invention can be implemented, at least in part, in computer-executable instructions, such as program modules, being stored on and executed by a computer, such as the above-mentioned server 1, the personal computer 3, and the portable device 6. Generally, program modules include routine programs, objects, components, data structures, etc., which perform particular tasks or implement particular abstract data types. Tasks performed by the program modules were described above.

Those skilled in the art can implement the description provided above to computer-executable instructions. In addition, those skilled in the art will appreciate that the invention may be practised with other computer system configurations, including multi-processor systems, networked personal computers, mini-computers, main frame computers, and the like. The invention may also be practised in distributed computing environments where tasks are performed by remote processing devices that are linked through a communications network. In a distributed computer environment, program modules and/or data may be located in both local and remote memory storage mediums.

The computers illustrated in the figure comprise a conventional computer having a central processing unit (CPU), memory and a system bus, which couples various system components, including the storage medium or media to the CPU. The system bus may be any of several types of bus structures, including a memory bus or a memory controller, a peripheral bus, a network bus and a local bus using any of a variety of bus architectures. A basic input/output system (BIOS) containing the basic routine that helps to transfer information between elements within the computer, such as during start-up, is preferably stored in ROM.

It should be appreciated by those skilled in the art that other types of computer readable media that are accessible by a computer, such as hard disks, floppy disk drives, optical disks, magnetic cassettes, flash memory cards, digital video disks, random access memories, read only memories, and the like, may also be used as storage media.

An input device such as a keyboard, pointing device (mouse), or the like, allows an operator to provide commands to the server 1, the personal computer 13, and , optionally, the portable device 6. A monitor or other type of output device is further connected to the system bus via a suitable interface and provides feedback to the operator. The invention is not in any way restricted to the above described embodiments and can be varied in many ways within the scope of the claims.

## Claims

1. A method of generating and/or adjusting a training schedule based on personal data and optionally on preferences of a particular user, which method involves electronically obtaining one or more parameters from and/or concerning the user during a training session preferably by means of at least one sensor, selecting and obtaining a training schedule from a database and/or adjusting an earlier selected schedule in accordance with the said obtained parameters.

2. The method of claim 1, wherein a plurality of parameters is obtained from a plurality of sensors.

3. The method of claim 2, wherein the parameters relate at least to the heart rate and the distance covered during a training session.

4. The method of any one of the preceding claims, wherein the database is improved on the basis of the parameters obtained from and/or the training schedules adjusted for a plurality of users.

5. The method of any one of the preceding claims, wherein the said parameters are uploaded to a remote service system, which selects and obtains a training schedule and/or adjusts an earlier selected schedule in accordance with the said parameters, and wherein the said schedule is subsequently downloaded.

6. A computer program product which carries out the method steps of any one of the preceding claims.

7. A computer program product stored on a computer readable storage medium comprising code means adapted to carry out the method steps of any one of the claims 1 to 5.

8. A portable device at least comprising:
an input device, such as preferably one or more sensors, for obtaining one or more parameters from or concerning a user during a training session,
a storage medium,
a controller adapted to store a training schedule and connected to the input device and the storage medium, the controller being operative to perform at least the following functions: receive the one or more parameters from the input device, store the one or more parameters in the storage medium,
an interface for transferring at least some of the stored information to a computer that contains or is connected or connectable to a database of training schedules.

9. The portable device of claim 8, which further comprises a display.

10. The portable device of claim 9, wherein the controller is further operative to analyse the course of the one or more parameters and to show results of this analysis in the display.

11. The portable device of any one of claims 8-10, wherein the controller is further operative to adjust the training schedule during a training session.

12. The portable device of any one of claims 8-11, wherein the said interface is operative to communicate wirelessly with the said computer.

13. A server system for generating and/or adjusting a training schedule based on personal data and optionally preferences of a particular user, an interface for receiving parameters from and/or concerning the user obtained during a training session, a storage medium for the said parameters, and a component for selecting and obtaining a training schedule from a database and/or adjusting an earlier selected schedule in accordance with the said obtained parameters.

14. The server system of claim 13, which comprises a further storage medium containing a database of training schedules.
